# EUROPÄISCHE PATENTSCHRIFT

(11) **EP 0 985 430 B1**
(45) Veröffentlichungstag und Bekanntmachung des Hinweises auf die Patenterteilung: **25.05.2005**
(21) Anmeldenummer: 99116180.3
(22) Anmeldetag: 24.08.1999
(51) Int. Cl.: A62B 9/00, A61M 16/08

(54) **Schalldämpfer für Beatmungsvorrichtungen und Inhalatoren**
Muffler for respiratory devices and inhalators
Silencieux pour dispositifs respiratoires et inhalateurs

(30) Priorität: 07.09.1998 DE 19840760
(43) Veröffentlichungstag der Anmeldung: 15.03.2000
(73) Patentinhaber: MAP Medizin-Technologie GmbH, 82152 Martinsried (DE)
(72) Erfinder: Lang, Bernd C., 82131 Gauting (DE)
(74) Vertreter: VOSSIUS & PARTNER

(56) Entgegenhaltungen:
- EP-A- 0 150 912
- EP-A- 0 467 362
- FR-A- 2 505 658
- GB-A- 2 095 121
- US-A- 3 856 051

## Beschreibung

Die vorliegende Erfindung betrifft einen Schalldämpfer für eine Beatmungsvorrichtung oder Inhalator mit einer Maske, Atemhilfsgeräten zur Bereitstellung von Atemluft, einem Schlauch zur Verbindung der Atemhilfsgeräte mit der Maske und mindestens einer Auslaßöffnung für Kohlendioxid.

Im Rahmen der Erfindung sind unter Beatmungsvorrichtungen Geräte zu verstehen, die die Atmung eines Patienten unterstützen oder völlig übernehmen. Herkömmliche Geräte bestehen dazu im wesentlichen aus einer Maske, die über den Mund und/oder die Nase eines Patienten gebracht wird, und einem Schlauch zur Verbindung der Maske mit einem Atemhilfsgerät (z.B. CPAP- und BiPAP-Atemhilfsgeräte), das zur Bereitstellung von Atemluft dient. Der Verbindungsschlauch ist flexibel und kann sowohl an der Maske als auch am Atemhilfsgerät drehbar befestigt sein.

Ferner sind Beatmungsvorrichtungen bekannt, bei denen zwischen der Maske und dem Schlauch ein Zwischenelement vorgesehen ist, das mit Schlitzen versehen ist, um das in der Atemluft enthaltene Kohlendioxid zumindest teilweise aus der Beatmungsvorrichtung zu entfernen. die Schlitze sind z.B. schräg zur Strömungsrichtung angeordnet und haben eine Breite von etwa 0,2 mm. Nachteilig bei der Ausführung solcher Schlitze ist vor allem, daß dadurch relativ laute Strömungsgeräusche entstehen, die Vorrichtung nur schwer reinigbar ist, der Patient unter Umständen Zugluft ausgesetzt ist, die aus den Schlitzen ausströmt, und die Einstellung eines bestimmten Ausströmwiderstands nur äußerst schwer möglich ist.

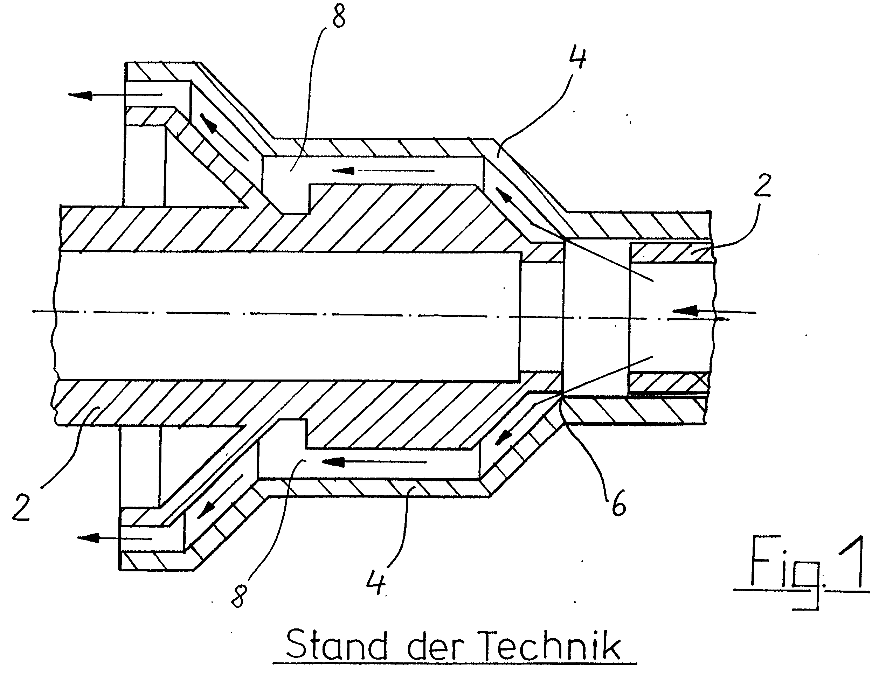

Des weiteren sind Beatmungsvorrichtungen mit einem Auswaschadapter mit spalt- oder lochförmigen, großen Strömungsquerschnitten bekannt. Der Druckabbau von Therapiedruck auf Umgebungsdruck findet in einem Zwischenelement statt, wie es in Fig. 1 dargestellt ist. Das Zwischenelement besteht im wesentlichen aus einem inneren Teilelement 2 und einem äußeren Teilelement 4. Der Druckabbau findet hauptsächlich in Strömungskanälen 6 mit geringer geometrischer Ausdehnung, d.h. Länge und geometrischer Verteilung, statt. Die im Anschluß an die Strömungskanäle 6 gebildeten Kanäle 8 dienen zur Luftleitung, jedoch nicht zum Druckabbau. Besonders nachteilig bei dieser Beatmungsvorrichtung mit dem oben beschriebenen Zwischenelement ist, daß durch die Ausbildung der Strömungskanäle 6 relativ laute Strömungsgeräusche entstehen und das Zwischenelement aufgrund seiner Bauweise nur schwierig demontierbar ist.

Daher liegt der vorliegenden Erfindung die Aufgabe zugrunde, einen verbesserten Schalldämpfer für Beatmungsvorrichtungen zur Verfügung zu stellen. Diese Aufgabe wird mit den Merkmalen der Patentansprüche gelöst.

Bei der Lösung dieser Aufgabe geht die Erfindung von dem Grundgedanken aus, in einem Beatmungsschlauch einer Beatmungsvorrichtung einen Schalldämpfer vorzusehen, der so ausgebildet ist, daß der Druckabbau über eine möglichst lange Strecke stattfindet, d.h. im wesentlichen über die gesamte Länge und den gesamten Umfang eines Ringkanals.

Die Beatmungsvorrichtung der vorliegenden Erfindung hat daher gegenüber dem Stand der Technik insbesondere die Vorteile, daß sie sehr leise arbeitet, d.h. geringe Strömungsgeräusche entstehen, leicht zu reinigen ist, eine schnelle Verbindung zwischen der Maske und dem Schlauch möglich ist, der Schlauch spannungsfrei geführt ist, indem er um seine Längsachse drehbar um einen Stutzen der Maske befestigbar ist und die ausströmende Luft automatisch vom Patienten weggeführt wird, so daß er keiner Zugluft ausgesetzt ist. Ein weiterer Vorteil besteht darin, daß der erfindungsgemäße Schalldämpfer hinsichtlich der Kondenswassercharakteristik unproblematisch ist.

Die Beatmungsvorrichtung der vorliegenden Erfindung wird im folgenden anhand einer bevorzugten Ausführungsform beispielhaft beschrieben. In den Zeichnungen zeigen:
- Fig. 1: ein in einem Beatmungsschlauch vorgesehenes Zwischen- oder Schalldämpferelement einer Beatmungsvorrichtung vom Stand der Technik;
- Fig. 2: eine Übersichtsdarstellung einer Beatmungsvorrichtung; und
- Fig. 3: einen erfindungsgemäßen Schalldämpfer für eine Beatmungsvorrichtung.

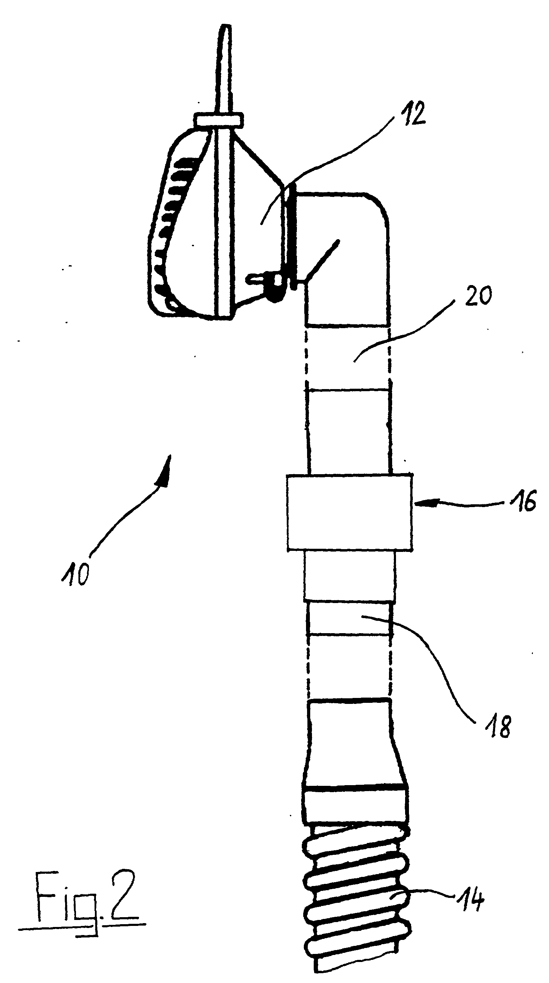

Die in Fig. 2 dargestellte Beatmungsvorrichtung 10 besteht im wesentlichen aus einer Maske 12, die über dem Mund und/oder der Nase eines Patienten befestigbar ist, (nicht dargestellten) Atemhilfsgeräten zur Bereitstellung von Atemluft für den Patienten, einem Schlauch 14 zur Verbindung der Atemhilfsgeräte mit der Maske 12 und einem Zwischenelement oder Schalldämpfer 16. Der Schalldämpfer 16 befindet sich zwischen einem der Maske 12 zugewandten Endabschnitt 18 des Schlauches 14 und einem von der Maske 12 abgewandten schlauch- oder rohrartigen Endabschnitt 20 der Maske 12. Vorzugsweise ist der Schalldämpfer 16 nahe der Maske 12 vorgesehen.

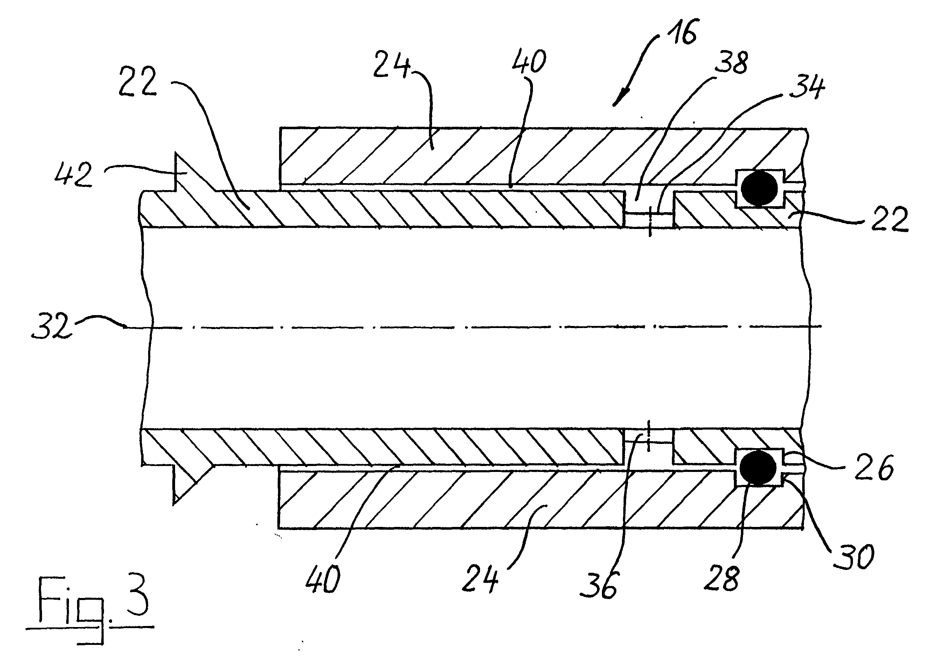

Der in Fig. 3 näher dargestellte Schalldämpfer 16 ist so ausgebildet, daß er zum "Auswaschen" d.h. zum Abführen des in der Atemluft enthaltenen Kohlendioxids geeignet ist, wobei gleichzeitig eine hohe Schalldämpfung bewirkt wird. Der Schalldämpfer 16 besteht im wesentlichen aus einem am Endabschnitt 18 des Schlauchs 14 angeordneten Innenrohr 22 und einem am Endabschnitt 20 der Maske 12 darüber angeordneten Außenrohr 24. Das Innenrohr 22 kann zusammen mit dem Endabschnitt 18 einteilig ausgebildet sein oder über geeignete Verbindungsmittel, vorzugsweise Schnellverbindungsmittel, damit verbindbar sein.

Vorzugsweise ist an dem der Maske 12 zugewandten Ende des Innenrohrs 22 eine umlaufende Außen-Ringnut 26 vorgesehen, in der ein Dichtungs- oder Schnappelement 28, vorzugsweise ein Federring oder O-Ring, eingefügt ist. Das Außenrohr 24 weist eine korrespondierende umlaufende Innen-Ringnut 30 auf, in die das Dichtungs- oder Schnappelement 28 eingreift. Das Dichtungselement 28 dient außer zur Fluidabdichtung ferner zur Körperschallentkopplung zwischen den maskenseitigen Elementen und den schlauchseitigen Elementen der Beatmungsvorrichtung. Weiterhin stellt das Dichtungselement 28 einen wichtigen Bestandteil eines Schnellverbindungsmittels dar, indem es das Innenrohr 22 mit dem Außenrohr 24 durch ihre jeweiligen Ringnuten 26 und 30 axial fixiert, jedoch Relativdrehungen der maskenseitigen Elemente und der schlauchseitigen Elemente um eine Rotationsachse 32 ermöglicht. Diese Ausgestaltung ermöglicht, daß der Beatmungsschlauch 14 bei Bewegungen des Patienten spannungsfrei gehalten wird, was eine wichtige Voraussetzung für einen guten Sitz der Maske 12 sein kann.

In der Nähe des stromaufwärtigen Endes des Innenrohrs 22 ist ferner mindestens eine radiale Öffnung 34 vorgesehen. Vorzugsweise ist jedoch eine Vielzahl radialer Öffnungen 34 um den Umfang verteilt vorgesehen, so daß das Innenrohr 22 an dieser Stelle lediglich durch wenige Verbindungsstege 36 zusammengehalten wird, um die für den Schalldämpfer 16 erforderliche Stabilität zu gewährleisten. Ferner sind diese Stege 36 vorzugsweise in ihrer Wandstärke, d.h. in der Differenz zwischen Außen- und Innendurchmesser, reduziert, so daß ein erster Ringraum 38 entsteht. In einer bevorzugten Ausführungsform der Erfindung sind um den Umfang des Innenrohrs 22 mit im wesentlichen gleichem Abstand zueinander 16 Öffnungen 34 vorgesehen, die jeweils einen Durchmesser von etwa 1,5 bis 2,5 mm aufweisen.

Das in der Ausatemluft enthaltene Kohlendioxid entweicht durch die radialen Öffnungen 34 in den Ringraum 38 und von dort durch einen zwischen dem Innenrohr 22 und dem Außenrohr 24 gebildeten zweiten Ringraum oder -kanal 40 in die Umgebung. Der zweite Ringkanal 40 weist vorzugsweise eine konstante Ringbreite, d.h. eine konstante Differenz zwischen Außen- und Innendurchmesser auf. Dieser zweite Ringkanal 40 ist vorzugsweise etwa 5 mm lang, hat einen Innendurchmesser von ca. 24 mm und eine Spaltbreite von ungefähr 0,3 mm. Dadurch erfolgt der Druckabbau der abgeleiteten Atemluft entlang der gesamten Länge des zweiten Ringkanals 40, wodurch dann die Geräuschemission des Schalldämpfers 16 bzw. der gesamten Beatmungsvorrichtung erheblich reduziert wird.

Eine Besonderheit des erfindungsgemäßen Schalldämpfers besteht darin, daß der Druckabbau überwiegend im Bereich des zweiten Ringkanals 40 erfolgt. Der erfindungsgemäße Schalldämpfer verursacht einen besonders geräuscharmen Leckagestrom, indem dem zweiten Ringkanal 40 (Druckabbau-Ringkanal) gleichmäßig Luft über den ersten Ringkanal 38 zugeführt wird. Bei den üblichen Druckgradienten entlang des zweiten Ringkanals 40 herrscht dabei aufgrund der besonderen Geometrie und Abmessungen des erfindungsgemäßen Schalldämpfers im Regelfall eine laminare Strömung.

Es ist insbesondere vorteilhaft, den erfindungsgemäßen Schaldämpfer so zu gestalten, daß die Summe der Querschnittsflächen der Durchgangskanäle bzw. Radialöffnungen 34 größer ist als die Querschnittsfläche des zweiten Ringkanals 40.

Vorzugsweise ist am strömungsabwärtigen Ende des Innenrohrs 22 eine umlaufende Leitnase 42 vorgesehen, die die ausströmende Ausatemluft über den aufgesteckten Schlauch vom Patienten weg leitet.

In einer weiteren Ausführungsform der Beatmungsvorrichtung (nicht dargestellt) ist am Innenrohr 22 und/oder am Außenrohr 24 mindestens ein Anschlußmittel vorgesehen, um Druckmessungen durchführen zu können und/oder weitere Fluide, wie z.B. Sauerstoff, dadurch bereitzustellen.

Ferner kann die Beatmungsvorrichtung in einer Ausführungsform (nicht dargestellt) Fluid-Leitmittel aufweisen, die je nach Strömungsrichtung im Zwischenelement bzw. Schalldämpfer 16 die radialen Öffnungen 34 beim Einatmen verschließen und sie beim Ausatmen wieder freigeben. Ferner kann die Fluidströmung in den Schlauch zurück zumindest behindert werden, so daß ein großer Teil des Kohlendioxids durch die radialen Öffnungen 34 über den ersten und zweiten Ringkanal 38 bzw. 34 entweichen kann, indem beim Ausatmen Fluid-Leitmittel den Strömungsweg zurück in den Schlauch 14 blockieren.

Wie vorstehend bereits erwähnt, ist es vorteilhaft, Relativbewegungen des Schlauches 14 zur Maske 12 zu ermöglichen. Dazu kann z.B. das Innenrohr 22 des Schalldämpfers 16 ein Ende des Schlauches 14 bilden oder daran angebracht sein. Das Außenrohr 24 kann dann beispielsweise am Endabschnitt 20 gebildet oder daran angebracht sein. Durch ein geeignetes Verschluß- oder Verbindungsmittel, vorzugsweise das zuvor beschriebene Schnellverschlußmittel kann ein schnelles Schließen und Öffnen der Verbindung sowie Relativdrehungen der Elemente zueinander gewährleistet werden. Es ist allerdings auch denkbar, den Schalldämpfer 16 so auszubilden, daß das Innenrohr 22 sowohl mit dem schlauchseitigen Endabschnitt 18 als auch mit dem maskenseitigen Endabschnitt 20 verbunden ist und das Außenrohr 24 nur zur Bildung des Ringkanals 40 vorgesehen ist. Zur Gewährleistung der Relativdrehung des Schlauches 14 zur Maske 12 können entsprechende Schnellverschlußmittel 26, 28 und 30 in diesem Fall zwischen dem Innenrohr 22 und mindestens einem der Endabschnitte 18 oder 20 angeordnet sein.

## Patentansprüche

1. Schalldämpfer (16) für eine Beatmungsvorrichtung (10), der ein mit einem Beatmungsschlauch (14) in Verbindung stehendes Innenrohr (22) und ein sich zumindest teilweise darüber erstreckendes Außenrohr (24) aufweist, wobei am Innenrohr (22) mindestens ein Durchgangskanal (34) vorgesehen ist, der sich in im wesentlichen radialer Richtung erstreckt und der über einen ersten Ringkanal (38) in einen sich zwischen dem Innenrohr (22) und Außenrohr (24) axial erstreckenden zweiten Ringkanal (40) mit im wesentlichen konstanter Ringbreite mündet, wobei der Innendurchmesser des ersten Ringkanals (38) kleiner ist als der des zweiten Ringkanals (40).

2. Schalldämpfer (16) nach Anspruch 1, wobei das Innenrohr (22) an einem schlauchseitigen Endabschnitt (18) und das Außenrohr an einem maskenseitigen Endabschnitt (20) vorgesehen ist.

3. Schalldämpfer (16) nach Anspruch 1 oder 2, wobei das Innenrohr (22) und das Außenrohr (24) als schnell zu schließende und zu öffnende Verbindung zwischen einer Maske (12) und dem Schlauch (14) ausgebildet sind.

4. Schalldämpfer (16) nach einem der Ansprüche 1 bis 3, mit einem Dichtungs- oder Schnappelement (28) zwischen dem Innenrohr (22) und dem Außenrohr (24).

5. Schalldämpfer (16) nach Anspruch 4, wobei das Dichtungsoder Schnappelement (28) in eine am Innenrohr (22) gebildete Außen-Ringnut (26) und in eine am Außenrohr (24) gebildete Innen-Ringnut (30) eingreift, um die schnell zu schließende und zu öffnende Verbindung und eine Abdichtung bereitzustellen.

6. Schalldämpfer (16) nach einem der Ansprüche 1 bis 5, wobei um den Umfang des Innenrohrs (22) mehrere Durchgangskanäle (34) angeordnet sind, die durch Stege (36) voneinander getrennt sind.

7. Schalldämpfer (16) nach einem der Ansprüche 1 bis 6, wobei am strömungsabwärtigen Ende des Innenrohrs (22), außerhalb der Umschließung durch das Außenrohr (24) eine Strömungsleitnase (42) vorgesehen ist.

8. Schalldämpfer (16) nach Anspruch 7, wobei die Strömungsleitnase (42) ringförmig ausgebildet ist.

9. Schalldämpfer (16) nach einem der Ansprüche 1 bis 8 mit mindestens einem Druckmeßanschluß, und/oder mindestens einem weiteren Anschluß zur Bereitstellung weiterer Behandlungsmittel.

10. Schalldämpfer (16) nach Anspruch 9, wobei die zusätzlichen Anschlüsse am Außenrohr (24), vorzugsweise am maskenseitigen Endabschnitt (20), vorgesehen sind.

11. Schalldämpfer (16) nach einem der Ansprüche 1 bis 10, wobei die Summe der Querschnittsflächen der Durchgangskanäle (34) größer als die Querschnittsfläche des zweiten Ringkanals (40) ist.

## Claims

1. A sound damper (16) for a respirator apparatus (10) having an inner tube (22) communicating with a respiration hose (14) and an outer tube (24) which at least partially extends over the inner tube, wherein at least one through passage (34) is provided on the inner tube (22) which through passage (34) extends in a substantially radial direction and opens by way of a first annular passage (38) into a second annular passage (40) of substantially constant annular width and extends axially between the inner tube (22) and the outer tube (24), wherein the inside diameter of the first annular passage (38) is smaller than that of the second annular passage (40).

2. A sound damper (16) according to claim 1 wherein the inner tube (22) is provided at an end portion (18) towards the hose and the outer tube is provided at an end portion (20) towards the mask.

3. A sound damper (16) according to claim 1 or claim 2 wherein the inner tube (22) and the outer tube (24) are in the form of a connection which is to be closed and opened quickly between a mask (12) and the hose (14).

4. A sound damper (16) according to one of claims 1 to 3 comprising a sealing or snap element (28) between the inner tube (22) and the outer tube (24).

5. A sound damper (16) according to claim 4 wherein the sealing or snap element (28) engages into an external annular groove (26) formed on the inner tube (22) and into an internal annular groove (30) formed on the outer tube (24) in order to provide a sealing integrity and the connection which is to be closed and opened quickly.

6. A sound damper (16) according to one of claims 1 to 5 wherein a plurality of through passages (34) separated from each other by limbs (36) are arranged around the periphery of the inner tube (22).

7. A sound damper (16) according to one of claims 1 to 6 wherein a flow guide projection (42) is provided at the downstream end of the inner tube (22) outside the enclosure by the outer tube (24).

8. A sound damper (16) according to claim 7 wherein the flow guide projection (42) is of an annular configuration.

9. A sound damper (16) according to one of claims 1 to 8 with at least one pressure measuring connection and/or at least one further connection for providing further treatment means.

10. A sound damper (16) according to claim 9 wherein the additional connections are provided on the outer tube (24), preferably at the end portion (20) towards the mask.

11. A sound damper (16) according to one of claims 1 to 10 wherein the sum of the cross-sectional areas of the through passages (34) is greater than the cross-sectional area of the second annular passage (40).

## Revendications

1. Silencieux (16) pour un dispositif respiratoire (10) qui présente un tuyau intérieur (22) qui est en liaison avec un tuyau flexible respiratoire (14) et un tuyau extérieur (24) s'étendant au moins partiellement par-dessus le tuyau intérieur, au tuyau intérieur (22) au moins un canal de passage (34) étant prévu qui s'étend essentiellement en direction radiale et qui débouche par l'intermédiaire d'un premier canal annulaire (38) dans un second canal annulaire (40) s'étendant axialement entre le tuyau intérieur (22) et le tuyau extérieur (24) et présentant une largeur d'anneau essentiellement constante, le diamètre intérieur du premier canal annulaire (38) étant plus petit que celui du second canal annulaire (40).

2. Silencieux (16) selon la revendication 1, le tuyau intérieur (22) étant prévu sur une section d'extrémité (18) côté tuyau flexible et le tuyau extérieur sur une section d'extrémité (20) côté masque.

3. Silencieux (16) selon les revendications 1 ou 2, le tuyau intérieur (22) et le tuyau extérieur (24) étant formés pour un raccordement se fermant et s'ouvrant rapidement entre un masque (12) et le tuyau flexible (14).

4. Silencieux (16) selon l'une quelconque des revendications 1 à 3, présentant un élément d'étanchéité ou d'encliquetage (28) entre le tuyau intérieur (22) et la tuyau extérieur (24).

5. Silencieux (16) selon la revendication 4, l'élément d'étanchéité ou d'encliquetage (28) s'engageant dans une rainure annulaire extérieure (26) prévue au tuyau intérieur (22) et une rainure annulaire intérieure (30) prévue au tuyau extérieur (24) pour mettre à disposition un raccordement fermant et ouvrant rapidement et une étanchéité.

6. Silencieux (16) selon l'une quelconque des revendications 1 à 5, plusieurs canaux de passage (34) étant prévus autour de la circonférence du tuyau intérieur (22), qui sont séparés l'un de l'autre par des nervures (36).

7. Silencieux (16) selon l'une quelconque des revendications 1 à 7, un nez déflecteur du flux (42) étant prévu à l'extrémité en aval du flux du tuyau intérieur (22) à l'extérieur de l'encerclement par le tuyau extérieur (24).

8. Silencieux (16) selon la revendication 7, le nez déflecteur du flux (42) présentant une forme annulaire.

9. Silencieux (16) selon l'une quelconque des revendications 1 à 8, comportant au moins un raccordement de mesure de la pression et/ou au moins un raccordement supplémentaire pour la mise à disposition de moyens de traitement supplémentaires.

10. Silencieux (16) selon la revendication 9, les raccordements supplémentaires étant prévus au tuyau extérieur (24), de préférence dans la section d'extrémité (20) côté masque.

11. Silencieux (16) selon l'une quelconque des revendications 1 à 10, la somme des superficies transversales des canaux de passage (34) étant plus grande que la superficie transversale du second canal annulaire (40).
